# EUROPEAN PATENT SPECIFICATION

(11) **EP 0 655 436 B1**
(45) Date of publication and mention of the grant of the patent: **11.03.1998**
(21) Application number: 94118234.7
(22) Date of filing: 18.11.1994
(51) Int. Cl.: C07C 221/00, C07C 225/22

(54) **Improved process for the manufacture of cycloalkyl and haloalkyl o-aminophenyl ketones**
Verfahren zur Herstellung von Cycloalkyl und Haloalkyl O-Amino-Phenylketonen
Procédé pour la préparation de cycloalkyl et haloalkyl o-aminophényl cétones

(30) Priority: 30.11.1993 US 159984; 30.12.1993 US 175823
(43) Date of publication of application: 31.05.1995
(73) Proprietor: AMERICAN CYANAMID COMPANY, Wayne, NJ 07470-8426 (US)
(72) Inventor: Cevasco, Albert Anthony, Mercer County, NJ 08502 (US)
(74) Representative: Wächtershäuser, Günter, Prof. Dr.

(56) References cited:
- EP-A- 0 463 287
- EP-A- 0 577 945
- DD-A- 249 608
- US-A- 4 160 784
- US-A- 4 988 695

## Description

Cycloalkyl and haloalkyl o-aminophenyl ketones are utilized as starting materials in the manu-facture of herbicidal sulfamoyl urea derivatives. The regiospecific preparation of o-aminophenyl ketone deriva-tives is described in US-A-4 160 784. Said patent provides a broad inclusive method to prepare a wide variety of aromatic and nonaromatic, substituted and unsubstituted o-aminophenyl ketones. However, in many instances the formation of unwanted side-products can cause decreased yields and cumbersome product isolation procedures.

DD249208 describes a process for producing 4-fluorophenyl cyclopropyl ketone by reacting fluorobenzene with gamma-chlorobutyryl chloride in the presence of aluminum chloride in methylene chloride, decomposing the acylation complex with water, removing the aqueous phase and cyclizing the 4-fluoro-gamma-chlorobutyrophenone with aqueous alkaline in a two-phase aqueous-organic system in the presence of a phase transfer catalyst.

The compound o-aminophenyl cyclopropyl ketone is particularly useful in the manufacture of 1-(o-cyclopropylcarbonyl)phenylsulfamoyl urea derivatives which are highly potent, yet environmentally benign, cereal crop tolerant herbicides. Methods known to prepare o-aminophenyl cyclopropyl ketone such as those described in US-A-4 160 784 and US-A-5 009 699 use cyclopropylnitrile as starting material. However, cyclopropylnitrile is not commercially nor readily obtainable, whereas 4-chlorobutyronitrile is obtainable commercially. However, there is no effective method available to prepare o-aminophenyl cyclopropyl ketone from its 4-halo acyclic precursor, 1-(o-aminophenyl)-4-halo-1-butanone.

Therefore, it is an object of this invention to provide an improved process for the preparation of cycloalkyl and haloalkyl o-aminophenyl ketones which gives significantly reduced side-product formation and increased product yield.

It is a further object of this invention to provide an effective process for the preparation of o-aminophenyl cyclopropyl ketone via the dehydrohalogenation of 1-(o-aminophenyl)-4-halo-1-butanone.

It is a further object of this invention to provide an economic and convenient source of starting materials for the manufacture of herbicidal sulfamoyl urea derivatives.

The present invention provides an improved process for the manufacture of an o-aminophenyl ketone of formula I 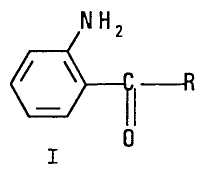 wherein R is C₃-C₆cycloalkyl or C₁-C₆haloalkyl, which comprises the following steps:
(1) reacting a nitrile of formula II

   R-CN II

   with borontrihalide in the presence of an organic solvent selected from the group consisting of halogenated hydrocarbons, aromatic hydrocarbons and halogenated aromatic hydrocarbons to form a 1:1 donor complex,
(2) reacting the complex with aniline in the presence of a Lewis acid to give a reaction mixture,
(3) sparging the reaction mixture with an inert gas such as nitrogen at a temperature of from 30° to 150°C for a period of 1-24 hours and
(4) quenching the sparged reaction mixture with water to give the formula I product.

The sequential process is shown in Flow Diagram I wherein R is C₃-C₆cycloalkyl or C₁-C₆haloalkyl and X is chlorine or bromine.

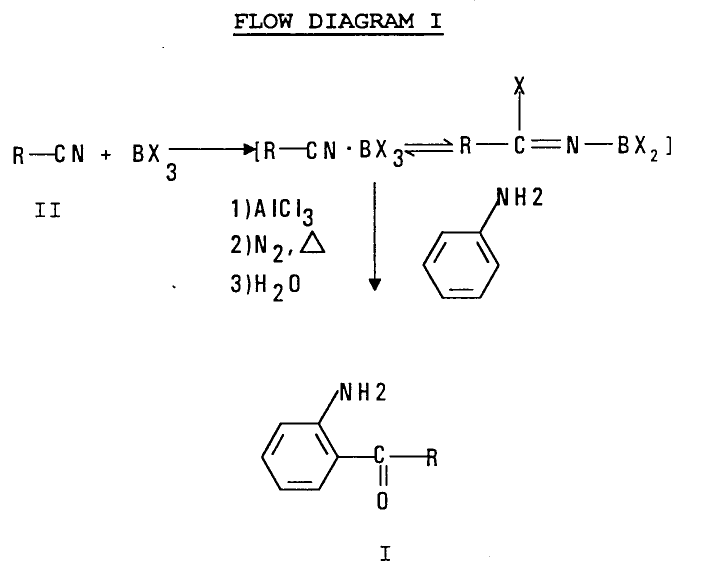

Advantageously, the 1:1 donor complex intermediate is not isolated, but is formed in situ allowing the process to be carried out in a single reaction vessel using a common solvent system. Surprisingly, the initial formation of the 1:1 donor complex of the nitrile and borontrihalide followed by the sequential addition of aniline and a Lewis acid avoids the formation of unwanted side-products which may tie up the aniline starting material and render it unavailable to form reaction product. The introduction of the nitrogen sparge to facilitate the removal of the hydrogen chloride by-product further enhances product yield and purity by decreasing, or eliminating, acid-catalyzed side reactions. It has now been found, that on a commercial scale an external force is critical to the effective removal of the hydrogen chloride gas by-product so that side reactions and reactant degradation are avoided.

Solvents used in the process of the invention are organic solvents such as halogenated hydrocarbons, for instance methylene chloride, dichloroethane, dichloropropane and the like; aromatic hydrocarbons such as toluene, xylene, benzene and the like; halogenated aromatics such as chlorobenzene, dichlorobenzene and the like. Preferred solvents are dichloroethane, dichloropropane and toluene and the more preferred solvent is dichloroethane.

The preferred Lewis acid for use in the inventive process is aluminum chloride.

The nitrogen sparge accelerates the loss of hydrogen chloride gas and helps to drive the reaction to completion. This acceleration, and the use of an inert gas such as nitrogen, appears to be critical to enhanced product yield and quality. Reaction time during the nitrogen sparge may range from 1-24 hours, preferably 8-16 hours and more preferably 12 hours.

Reaction rate increases with increased temperatures, however increased temperatures also increase reactant degradation and other undesirable side-reactions. Temperatures for the nitrogen sparge time period are 30° to 150°C, preferably 80° to 110°C.

Although stoichiometric proportions of reactants are suitable, a preferred range for the proportion of a nitrile of formula II to one molar equivalent of aniline is 1 to 2 moles and more preferred is 1.3 to 1.5 moles.

The present invention further provides a process for the preparation of o-aminophenyl cyclopropyl ketone wherein a compound of formula I' obtained according to Claim 1: 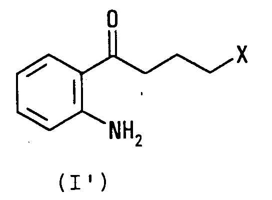 wherein X is chlorine or bromine is reacted with at least one molar equivalent of an aqueous base in the presence of a phase transfer catalyst and optionally in the presence of an organic solvent.

O-aminophenyl cyclopropyl ketone may be effectively prepared with minimal side-product formation by the dehydrohalogenation of 1-(o-aminophenyl)-4-halo-1-butanone using at least one molar equivalent, preferably 1.1 - 3.0 molar equivalents, of an aqueous base in the presence of a phase transfer catalyst and optionally in the presence of an organic solvent.

Although the dehydrohalogenation of a γ-haloalkyl ketone using aqueous base is known to form the corresponding cycloalkyl ketone, for example, as described in Organic Synthesis, Coll. Vol. 4, pp. 597-600 (1963), when these procedures are applied to 4-halobutanone compounds of formula I wherein X is chlorine or bromine, the reaction proceeds slowly and the major product is mainly the 4-hydroxy analogue of formula III.

Surprisingly, it has been found that when a phase transfer catalyst (PTC) is present in the reaction mixture, the reaction rate is effectively increased and the major product is the desired cyclopropyl ketone of formula I''. The reaction scheme is shown in flow diagram II.

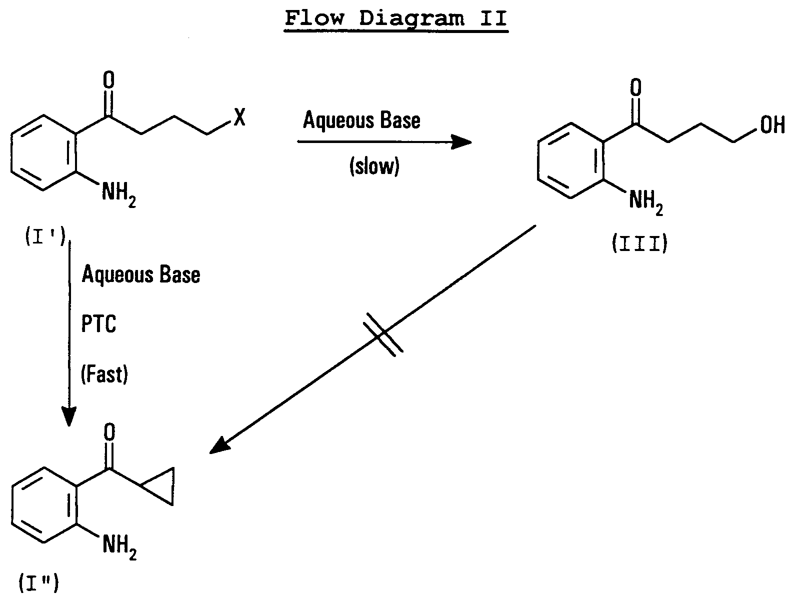

Phase transfer catalysts suitable for use in the inventive process are those well known in the art such as, quaternary ammonium salts, for example trialkyl ammonium salts or tetralkylammonium salts, preferably tri- or tetrabutylammonium halides. An effective amount of the catalyst may range from 0.001 - 0.50 molar equivalents.

Aqueous bases suitable for the preparation of o-aminophenyl cyclopropyl ketone by the present process include sodium and potassium hydroxide, carbonate or bicarbonate or mixtures thereof at concentrations in aqueous solution of 15% to 50% by weight in amounts sufficient to provide at least one molar equivalent, preferably 1.1 to 3.0 molar equivalents, and more preferably 1.5 to 2.5 molar equivalents.

The organic solvent may be any inert water immiscible solvent or mixtures of solvents such as halogenated hydrocarbons, alkyl and aromatic ethers, aromatic hydrocarbons, halogenated aromatic hydrocarbons, and the like, preferably halogenated hydrocarbons such as methylene dichloride, ethylene dichloride, propylene dichloride and the like.

In accordance with the process of the invention, a 4-halobutanone compound of formula I', optionally dissolved in an organic solvent or mixture of solvents, is admixed with at least one molar equivalent of an aqueous base, preferably 1.1 - 3.0 molar equivalents, more preferably 1.5 - 2.5 molar equivalents and a quaternary ammonium phase transfer catalyst, preferably methyl tributylammonium chloride, in an amount of 0.001 - 0.50 molar equivalents, preferably 0.025 - 0.50 molar equivalents.

For a more clear understanding of the invention, specific examples thereof are set forth below. These examples are merely illustrative and are not to be understood as limiting the scope and underlying principles of the invention in any way.

The terms HPLC and GC designates high performance liquid chromatography and gas chromatography, respectively.

### EXAMPLE 1

### PREPARATION OF o-AMINOPHENYL CYCLOPROPYL KETONE

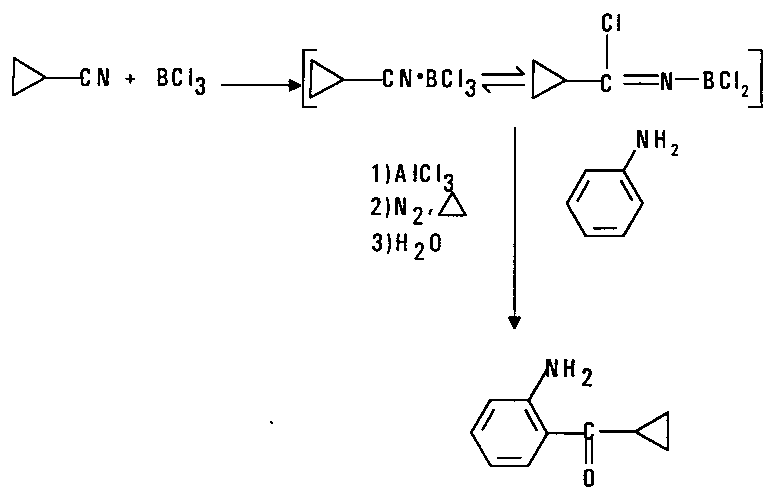

A stirred mixture of BCl₃ (145.2g, 1.238 mole) in dichloroethane is treated with cyclopropylnitrile (101.8g, 1.45 mole) over a 45 minute period at -3° to 5°C, stirred for 1 hour at 5° to 10°C, treated with aniline (112.2g, 1.205 mole) over a 1 hour period at 7° to 12°C, stirred for 1 hour, treated with AlCl₃(170.7g, 1.281 mole) in a single portion and stirred at ambient temperatures for 1 hour. The reaction mixture is sparged with nitrogen and heated at reflux temperatures with a nitrogen sparge for 17-18 hours. The heated and sparged reaction mixture is cooled to 15°C, added to approximately a 2-fold volume of water (based upon initial reaction mixture volume) over a 30 minute period at 9° to 38°C and stirred at 35°to 38° for 1 hour. The phases are separated and the aqueous phase is extracted with methylene chloride. The organic phases are combined and concentrated in vacuo to give the title product as an orange oil which crystallizes on standing, 149.8g, 95.6% pure, 73.7% yield, identified by HPLC analysis.

### EXAMPLE 2

### PREPARATION OF 1-(o-AMINOPHENYL)-4-CHLORO-1-BUTANONE

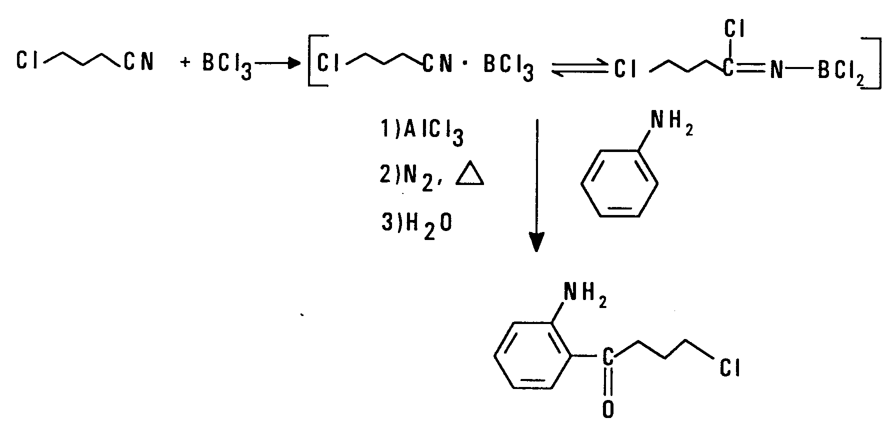

A mixture of BCl₃(59.2g, 0.50 mole) in 1,2-dichloroethane is treated with 4-chlorobutyronitrile (62.5g, 0.604 mole) over a 1 hour period at -8° to 0°C, stirred for 1 hour at 0°to 5°C, treated with aniline (45.1g, 0.485 mole) over a 1 hour period at 2° to 9°C, stirred for 1 hour, and treated with AlCl₃(68.8g, 0.515 mole) in a single portion at ambient temperatures. The reaction mixture is sparged with nitrogen at reflux temperature for about 17 hours. The heated and sparged reaction mixture is cooled to 35°C and added to approximately a 2-fold volume of water (based upon initial reaction volume) and stirred for 0.5 hour at 33° to 35°C. The phases are separated and the aqueous phase is washed with 1,2-dichloroethane. The organic phases are combined and concentrated in vacuo to give a solution of the title product containing, 85.9g of the desired o-aminophenyl ketone, 89.7% yield, identified by HPLC analysis.

### EXAMPLE 3

### COMPARATIVE PREPARATION OF 1-(o-AMINOPHENYL) CYCLOPROPYL KETONE

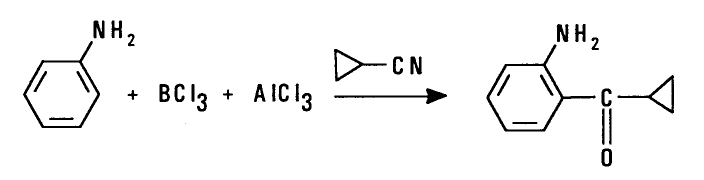

The procedure used in this example is essentially the same as that described in U. S. Patent 4,160,784.

A solution of aniline (2.7g, 0.029 mole) in 1,2-dichloroethane is treated with BCl₃ (3.40g, 0.029 mole) over a 20 minute period at -8° to 5°C, stirred at -2° to 5°C for 35 minutes, treated with cyclopropylnitrile (2.9g, 0.043 mole) over a 15 minute period at 0° to 5°C, treated with AlCl₃ (4.2g, 0.0315 mole) in a single portion and stirred at ambient temperatures for 1.5 hours. The reaction mixture is heated at reflux temperature for 18 hours, added to an excess volume of water and stirred for 16 hours. The phases are separated and the aqueous phase is extracted with methylene chloride. The organic phases are combined and concentrated in vacuo to give the title product, 2.55g, 90% pure, 49.1% yield, identified by HPLC analysis.

### EXAMPLE 4

### COMPARATIVE PREPARATION OF 1-(o-AMINOPHENYL)-4-CHLORO-1-BUTANONE

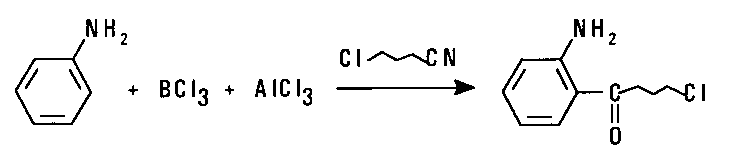

The following procedure is described in U. S. Patent 4,988,695.

To a solution of boron trichloride (55 g) in 1,2-dichloroethane (200 ml) at 0° was added dropwise a solution of aniline (39.1 g, 0.42 mol) in 1,2-dichloroethane (50 ml) maintaining the temperature below 5°. On completion of the addition 4-chlorobutyronitrile (41.4 g, 0.42 mol) and aluminium chloride (53,34 g, 0.42 mol) were added successively and the reaction mixture allowed to warm up to room temperature then heated under reflux for 20 hours. After allowing to cool, 2N hydrochloric acid (100 ml) was added and the reaction mixture heated under reflux for 0.5 hour. The solid obtained was collected by filtration, taken up in water and extracted with chloroform (5 X 200 ml). The chloroform extracts were combined, washed with dilute base, then water, dried over magnesium sulphate, filtered and evaporated under reduced pressure to give an oil. The oil was dissolved in ether and ethanolic HCl was added, the resulting solid was collected by filtration and dried, 28.5 g, 29% yield. Recrystallization from ethanol/diethylether gave the product hydrochloride salt, m.p. 152°-154°C.

### EXAMPLE 5

### Preparation of o-aminophenyl cyclopropyl ketone via the dehydrohalogenation of 1-(o-aminophenyl)-4-chloro-1-butanone in the presence of a phase transfer catalyst

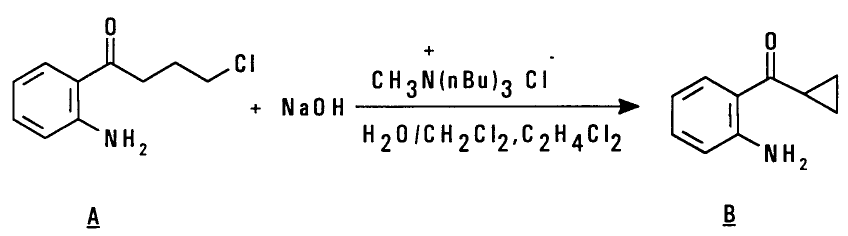

A solution of 1-(o-aminophenyl)-4-chloro-1-butanone (32.6g, 0.165 mole) in ethylenedichloride is treated with 147.3g of 20% aqueous NaOH (0.736 mole NaOH) and 2.31g of 75% aqueous methyl tributylammonium chloride(0.0074 mole), and stirred at 50°- 53°C for about 4 hours. (Samples are removed at intervals and analyzed by HPLC.) The reaction is cooled to room temperature and the phases are separated. The organic phase is washed with water and concentrated in vacuo to give the title product, 29.84g, 70.6% pure, 79.2% yield by HPLC and GC analyses.

Reaction progress is shown in Table I.

**Table I**

| **Sample** | **Time h** | **% A** | **% B** |
|---|---|---|---|
| 1 | 0.0 | 100.0 | 0.0 |
| 2 | 0.5 | 28.4 | 66.9 |
| 3 | 1.0 | 12.2 | 80.6 |
| 4 | 3.0 | 0.5 | 94.6 |
| 5 | 4.0 | 0.0 | 96.3 |

### EXAMPLE 6

### Evaluation of the effect of a phase transfer catalyst on the dehydrohalogenation of 1-(o-aminophenyl)-4-chloro-1-butanone

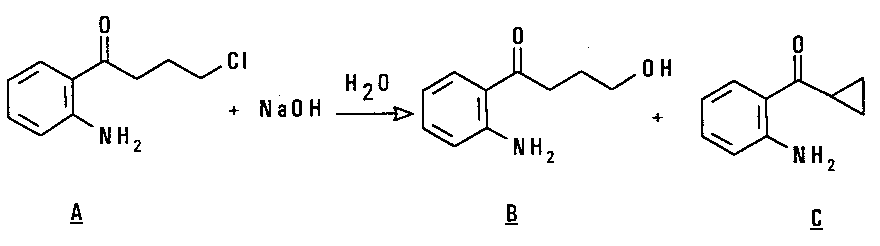

### GENERAL PROCEDURE

A mixture of 1-(o-aminophenyl)-4-chloro-1-butanone (35.6g, 0.18 mole) in a solvent mixture of ethylene dichloride and methylene dichloride and 146g of 20% aqueous NaOH (0.73 mole) is stirred at 50°- 85°C for 9 hours. After the 9 hour period, methyl tributylammonium chloride (1.74g, 0.0074 mole) is added and stirring is continued for an additional 0.5 hour. Aliquots of the reaction mixture are removed at 0.5 - 1 hour(h) time intervals and are assayed for the presence of starting material (A), 1-(o-aminophenyl)-4-hydroxy-1-butanone (B) and o-aminophenyl cyclopropyl ketone (C). The results are recorded and shown in Table II below.

The assays are performed using HPLC analysis.

**Table II**

| **Sample** | **Time h** | **Temp. °C** | **%A** | **%B** | **%C** |
|---|---|---|---|---|---|
| 1 | 0 | 50 | 92.0 | 1.0 | 0.0 |
| 2 | 0.5 | 50 | 85.6 | 0.9 | 0.5 |
| 3 | 1.0 | 50 | 89.0 | 1.5 | 1.8 |
| 4 | 2.0 | 50 | 89.4 | 2.2 | 0.8 |
| 5 | 3.0 | 50 | 91.2 | 3.8 | 1.1 |
| 6 | 4.0 | 50 | 83.0 | 3.8 | 1.0 |
| 7 | 5.0 | 67 | 86.4 | 6.9 | 1.5 |
| 8 | 6.0 | 67 | 83.0 | 7.8 | 1.7 |
| 9 | 7.0 | 67 | 78.0 | 14.1 | 3.2 |
| 10 | 8.0 | 76 | 73.0 | 19.2 | 3.5 |
| 11 | 9.0 | 85 | 51.8 | 33.5 | 7.1 |
| 12 | 9.5 | 85 | 0.0 | 34.3 | 56.0 |

As can be seen from Table II above, in the absence of a phase transfer catalyst (samples 1-11) the starting 4-chlorobutanone (A) is preferentially and slowly converted to the unwanted 4-hydroxybutanone by-product (B). However, 0.5 hour after the addition of the phase transfer catalyst (sample 12), the starting material is completely converted to, preferentially, the desired cyclopropyl ketone product (C). Hence, the reaction rate to the desired product (C) is about 250 times greater in the presence of a phase transfer catalyst than in the absence of a phase transfer catalyst.

## Claims

1. A process for the manufacture of an o-aminophenyl ketone of formula I 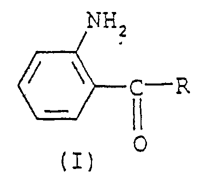 wherein R is C₃-C₆cycloalkyl or C₁-C₆haloalkyl which comprises the following steps:
(1) reacting a nitrile of formula II
R-CN (II)
with borontrihalide in the presence of a solvent to form 1:1 donor complex, whereby the solvent is selected from the group consisting of halogenated hydrocarbons, aromatic hydrocarbons and halogenated aromatic hydrocarbons,
(2) reacting the complex with aniline in the presence of a Lewis acid to give a reaction mixture,
(3) sparging the reaction mixture with an inert gas at a temperature of from 30° to 150°C for a period of 1-24 hours and
(4) quenching the sparged reaction mixture with water to give the formula I product.

2. The process according to Claim 1, wherein the solvent of step (1) is dichloropropane or dichloroethane, the borontrihalide is borontrichloride, the Lewis acid is aluminum chloride, the inert gas is nitrogen, the temperature is 30°C to 110°C and the sparging period is 8 to 16 hours.

3. The process according to Claim 2, wherein the solvent of step (1) is dichloroethane and the sparging period is 12 hours,

4. The process according to Claim 2, wherein the molar ratio of formula II compound to aniline is 1:1 to 2:1.

5. The process according to Claim 4, wherein the molar ratio of formula II compound to aniline is 1.3:1 to 1.5:1.

6. The process according to Claim 1, wherein the nitrile of formula II is cyclopropylnitrile.

7. The process according to Claim 1, wherein the nitrile of formula II is 4-halobutyronitrile.

8. The process according to Claim 7, wherein the 4-halobutyronitrile is 4-bromobutyronitrile or 4-chlorobutyronitrile or mixtures thereof.

9. The process according to Claim 1 for the preparation of a compound of formula I' 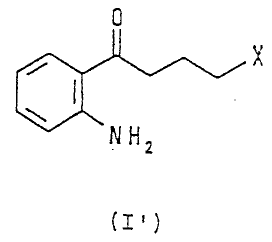 wherein X is chlorine or bromine characterized in that the process further comprises reacting the compound of formula I' with at least one molar equivalent of an aqueous base in the presence of a phase transfer catalyst and optionally in the presence of an organic solvent for preparing o-aminophenyl cyclopropyl ketone.

10. The process according to Claim 9, wherein the organic solvent is present and is ethylenedichloride, methylenedichloride, propylenedichloride or mixtures thereof, the aqueous base is NaOH or KOH and the phase transfer catalyst is a trialkylammonium halide or a tetraalkylammonium halide.

11. The process according to Claim 10, wherein X is chlorine.

12. The process according to Claim 10, wherein the aqueous base is present in an amount of 1.5 to 2.5 molar equivalents.

13. The process according to Claim 10, wherein the phase transfer catalyst is methyl tributylammonium chloride and the aqueous base is NaOH.

14. A process for the preparation of o-aminophenyl cyclopropyl ketone which comprises the following steps:
(1) reacting a 4-halobutyronitrile with a borontrihalide in the presence of a solvent selected from the group consisting of halogenated hydrocarbons, aromatic hydrocarbons and halogenated aromatic hydrocarbons, to form a 1:1 donor complex,
(2) reacting the complex with aniline in the presence of a Lewis acid to give a reaction mixture,
(3) sparging the reaction mixture with an inert gas at a temperature of from 30°C to 150°C for a period of 1-24 hours,
(4) quenching the sparged reaction mixture with water to give a compound of formula I' 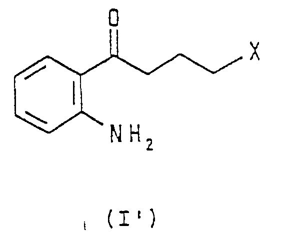 wherein X is chlorine or bromine,
and further reacting the formula I' compound with at least one molar equivalent of an aqueous base in the presence of a phase transfer catalyst and optionally in the presence of an organic solvent.

15. The process according to Claim 14, wherein the solvent of step (1) is dichloroethane or dichloropropane, the 4-halobutyronitrile is 4-bromobutyronitrile or 4-chlorobutyronitrile of mixtures thereof, the molar ratio of the 4-halobutyronitrile to aniline is 1:1 to 2:1, the borontrihalide is borontrichloride, the Lewis acid is aluminum chloride, the inert gas is nitrogen, the temperature is 30°C to 110°C and the sparging period is 8 to 16 hours.

16. The process according to Claim 15, wherein the organic solvent is present in step (4) and is ethylenedichloride, methylenedichloride, propylenedichloride or mixtures thereof, the aqueous base is NaOH or KOH and the phase transfer catalyst is methyl tributylammonium chloride.

## Patentansprüche

1. Verfahren zur Herstellung eines o-Aminophenylketons der Formel I 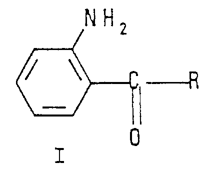 worin R C₃-C₆-Cycloalkyl oder C₁-C₆-Halogenalkyl darstellt, das die nachstehenden Schritte umfaßt:
(1) Umsetzen eines Nitrils der Formel II
R-CN (II)
mit einem Bortrihalogenid in Gegenwart eines Lösungsmittels zur Bildung eines 1:1 Donorkomplexes, wobei das Lösungsmittel ausgewählt ist aus der Gruppe, bestehend aus halogenierten Kohlenwasserstoffen, aromatischen Kohlenwasserstoffen und halogenierten aromatischen Kohlenwasserstoffen,
(2) Umsetzen des Komplexes mit Anilin in Gegenwart einer Lewis-Säure zu einem Reaktionsgemisch,
(3) Durchspülen des Reaktionsgemisches mit einem Inertgas bei einer Temperatur von 30° bis 150°C für einen Zeitraum von 1-24 Stunden und
(4) Stoppen des durchspülten Reaktionsgemisches mit Wasser unter Gewinnung des Produkts der Formel I.

2. Verfahren nach Anspruch 1, wobei das Lösungsmittel von Schritt (1) Dichlorpropan oder Dichlorethan darstellt, das Bortrihalogenid Bortrichlorid darstellt, die Lewis-Säure Aluminiumchlorid darstellt, das Inertgas Stickstoff darstellt, die Temperatur 30°C bis 110°C ist und der Durchspülzeitraum 8 bis 16 Stunden ist.

3. Verfahren nach Anspruch 2, wobei das Lösungsmittel von Schritt (1) Dichlorethan darstellt und der Durchspülzeitraum 12 Stunden ist.

4. Verfahren nach Anspruch 2, wobei das Molverhältnis der Verbindung der Formel II zu Anilin 1:1 bis 2:1 ist.

5. Verfahren nach Anspruch 4, wobei das Molverhältnis der Verbindung der Formel II zu Anilin 1,3:1 bis 1,5:1 ist.

6. Verfahren nach Anspruch 1, wobei das Nitril der Formel II Cyclopropylnitril ist.

7. Verfahren nach Anspruch 1, wobei das Nitril der Formel II 4-Halogenbutyronitril ist.

8. Verfahren nach Anspruch 7, wobei das 4-Halogenbutyronitril 4-Brombutyronitril oder 4-Chlorbutyronitril oder Gemische davon ist.

9. Verfahren nach Anspruch 1 zur Herstellung einer Verbindung der Formel I' 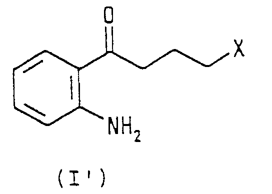 worin X Chlor oder Brom darstellt, dadurch gekennzeichnet, daß das Verfahren weiterhin umfaßt Umsetzen der Verbindung der Formel I' mit mindestens einem Moläquivalent einer wässerigen Base in Gegenwart eines Phasentransferkatalysators und gegebenenfalls in Gegenwart eines organischen Lösungsmittels unter Herstellung von o-Aminophenylcyclopropylketon.

10. Verfahren nach Anspruch 9, wobei das organische Lösungsmittel vorliegt und Ethylendichlorid, Methylendichlorid, Propylendichlorid oder Gemische davon darstellt, die wässerige Base NaOH oder KOH darstellt und der Phasentransferkatalysator ein Trialkylammoniumhalogenid oder ein Tetraalkylammoniumhalogenid darstellt.

11. Verfahren nach Anspruch 10, wobei X Chlor darstellt.

12. Verfahren nach Anspruch 10, wobei die wässerige Base in einer Menge von 1,5 bis 2,5 Moläquivalenten vorliegt.

13. Verfahren nach Anspruch 10, wobei der Phasentransferkatalysator Methyltributylammoniumchlorid darstellt und die wässerige Base NaOH darstellt.

14. Verfahren zur Herstellung von o-Aminophenylcyclopropylketon, das die nachstehenden Schritte umfaßt:
(1) Umsetzen eines 4-Halogenbutyronitrils mit einem Bortrihalogenid in Gegenwart eines Lösungsmittels, ausgewählt aus der Gruppe, bestehend aus halogenierten Kohlenwasserstoffen, aromatischen Kohlenwasserstoffen und halogenierten aromatischen Kohlenwasserstoffen, zur Bildung eines 1:1 Donorkomplexes,
(2) Umsetzen des Komplexes mit Anilin in Gegenwart einer Lewis-Säure zu einem Reaktionsgemisch,
(3) Durchspülen des Reaktionsgemisches mit einem Inertgas bei einer Temperatur von 30°C bis 150°C für einen Zeitraum von 1-24 Stunden,
(4) Stoppen des gespülten Reaktionsgemisches mit Wasser unter Gewinnung einer Verbindung der Formel I' 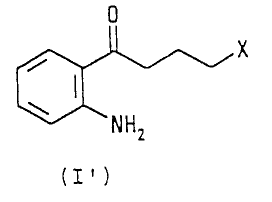 worin X Chlor oder Brom darstellt,
und weiterhin Umsetzen der Verbindung der Formel I' mit mindestens einem Moläquivalent einer wässerigen Base in Gegenwart eines Phasentransferkatalysators und gegebenenfalls in Gegenwart eines organischen Lösungsmittels.

15. Verfahren nach Anspruch 14, wobei das Lösungsmittel von Schritt (1) Dichlorethan oder Dichlorpropan darstellt, das 4-Halogenbutyronitril 4-Brombutyronitril oder 4-Chlorbutyronitril oder Gemische davon darstellt, das Molverhältnis des 4-Halogenbutyronitrils zu Anilin 1:1 bis 2:1 ist, das Bortrihalogenid Bortrichlorid darstellt, die Lewis-Säure Aluminiumchlorid darstellt, das Inertgas Stickstoff darstellt, die Temperatur 30°C bis 110°C ist und der Zeitraum für das Durchspülen 8 bis 16 Stunden ist.

16. Verfahren nach Anspruch 15, wobei das organische Lösungsmittel in Schritt (4) vorliegt und Ethylendichlorid, Methylendichlorid, Propylendichlorid oder Gemische davon darstellt, die wässerige Base NaOH oder KOH ist und der Phasentransferkatalysator Methyltributylammoniumchlorid darstellt.

## Revendications

1. Procédé pour la préparation d'une o-aminophénylcétone répondant à la formule I 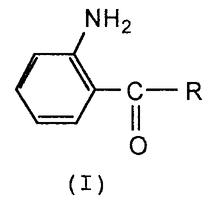 dans laquelle R représente un groupe cycloalkyle en C₃-C₆ ou halogénoalkyle en C₁-C₆, comprenant les étapes consistant à :
(1) faire réagir un nitrile de formule II
R-CN (II)
avec un trihalogénure de bore en présence d'un solvant, pour former un complexe donneur 1:1, le solvant étant choisi dans le groupe constitué des hydrocarbures halogénés, des hydrocarbures aromatiques et des hydrocarbures aromatiques halogénés,
(2) faire réagir le complexe avec de l'aniline en présence d'un acide de Lewis, pour obtenir un mélange réactionnel,
(3) faire barboter dans le mélange réactionnel un gaz inerte à une température de 30°C à 150°C pendant une durée de 1 à 24 heures, et
(4) arrêter la réaction du mélange réactionnel ayant subi le barbotage, avec de l'eau, pour obtenir le produit de formule I.

2. Procédé selon la revendication 1, dans lequel le solvant de l'étape (1) est le dichloropropane ou le dichloroéthane, le trihalogénure de bore est le trichlorure de bore, l'acide de Lewis est le chlorure d'aluminium, le gaz inerte est l'azote, la température est comprise entre 30°C et 110°C et la durée du barbotage est de 8 à 16 heures.

3. Procédé selon la revendication 2, dans lequel le solvant de l'étape (1) est le dichloroéthane et la durée du barbotage est de 12 heures.

4. Procédé selon la revendication 2, dans lequel le rapport molaire du composé de formule II à l'aniline vaut de 1:1 à 2:1.

5. Procédé selon la revendication 4, dans lequel le rapport molaire du composé de formule II à l'aniline vaut de 1,3:1 à 1,5:1.

6. Procédé selon la revendication 1, dans lequel le nitrile de formule II est le cyclopropylnitrile.

7. Procédé selon la revendication 1, dans lequel le nitrile de formule II est le 4-halogénobutyronitrile.

8. Procédé selon la revendication 7, dans lequel le 4-halogénobutyronitrile est le 4-bromobutyronitrile ou le 4-chlorobutyronitrile ou un de leurs mélanges.

9. Procédé selon la revendication 1 pour la préparation d'un composé répondant à la formule I' 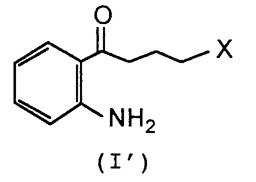 dans laquelle X représente un atome de chlore ou de brome, caractérisé en ce que le procédé comprend en outre la mise en réaction du composé de formule I' avec au moins un équivalent molaire d'une base aqueuse en présence d'un catalyseur de transfert de phase et éventuellement en présence d'un solvant organique, pour préparer de l'o-aminophényl-cyclopropylcétone.

10. Procédé selon la revendication 9, dans lequel le solvant organique est présent et consiste en dichloroéthane, dichlorométhane, dichloropropane ou un de leurs mélanges, la base aqueuse est NaOH ou KOH et le catalyseur de transfert de phase est un halogénure de trialkylammonium ou un halogénure de tétraalkylammonium.

11. Procédé selon la revendication 10, dans lequel X représente un atome de chlore.

12. Procédé selon la revendication 10, dans lequel la base aqueuse est présente en une quantité de 1,5 à 2,5 équivalents molaires.

13. Procédé selon la revendication 10, dans lequel le catalyseur de transfert de phase est le chlorure de méthyltributylammonium et la base aqueuse est NaOH.

14. Procédé pour la préparation d'o-aminophényl-cyclopropylcétone, comprenant les étapes consistant à :
(1) faire réagir un 4-halogénobutyronitrile avec un trihalogénure de bore en présence d'un solvant choisi dans le groupe constitué des hydrocarbures halogénés, des hydrocarbures aromatiques et des hydrocarbures aromatiques halogénés, pour former un complexe donneur 1:1,
(2) faire réagir le complexe avec de l'aniline en présence d'un acide de Lewis, pour obtenir un mélange réactionnel,
(3) faire barboter dans le mélange réactionnel un gaz inerte à une température de 30°C à 150°C pendant une durée de 1 à 24 heures,
(4) arrêter la réaction du mélange réactionnel ayant subi le barbotage, avec de l'eau, pour obtenir un composé de formule I' 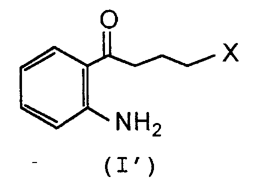 dans laquelle X représente un atome de chlore ou de brome, et à faire ensuite réagir le composé de formule I' avec au moins un équivalent molaire d'une base aqueuse en présence d'un catalyseur de transfert de phase et éventuellement en présence d'un solvant organique.

15. Procédé selon la revendication 14, dans lequel le solvant de l'étape (1) est le dichloroéthane ou le dichloropropane, le 4-halogénobutyronitrile est le 4-bromobutyronitrile ou le 4-chlorobutyronitrile ou un de leurs mélanges, le rapport molaire du 4-halogénobutyronitrile à l'aniline vaut de 1:1 à 2:1, le trihalogénure de bore est le trichlorure de bore, l'acide de Lewis est le chlorure d'aluminium, le gaz inerte est l'azote, la température est comprise entre 30°C et 110°C et la durée du barbotage est de 8 à 16 heures.

16. Procédé selon la revendication 15, dans lequel le solvant organique est présent dans l'étape (4) et consiste en dichloroéthane, dichlorométhane, dichloropropane ou un de leurs mélanges, la base aqueuse est NaOH ou KOH et le catalyseur de transfert de phase est le chlorure de méthyltributylammonium.
